(19) **Europäisches Patentamt**
**European Patent Office**
**Office européen des brevets**

(11) Veröffentlichungsnummer: **0 439 042 B1**

(12) # EUROPÄISCHE PATENTSCHRIFT

(45) Veröffentlichungstag der Patentschrift: **28.06.95**

(51) Int. Cl.[6]: **A61K 7/48**, A61K 9/107,
A61K 47/24, A61K 47/28

(21) Anmeldenummer: **91100372.1**

(22) Anmeldetag: **14.01.91**

(54) **Verwendung von pharmazeutischen und kosmetischen Präparaten mit Mischmicellen.**

(30) Priorität: **24.01.90 CH 222/90**

(43) Veröffentlichungstag der Anmeldung:
**31.07.91 Patentblatt 91/31**

(45) Bekanntmachung des Hinweises auf die
Patenterteilung:
**28.06.95 Patentblatt 95/26**

(84) Benannte Vertragsstaaten:
**AT BE CH DE DK FR GB IT LI LU NL SE**

(56) Entgegenhaltungen:
**EP-A- 0 229 561**
**WO-A-90/08534**
**FR-A- 2 423 219**
**US-A- 4 115 313**

(73) Patentinhaber: **F. HOFFMANN-LA ROCHE &
CO. Aktiengesellschaft
Postfach 3255
CH-4002 Basel (CH)**

(72) Erfinder: **Pittrof, Folker, Dr.
Hintern Weiher
W-7800 Freiburg 36 (DE)**
Erfinder: **Supersaxo, Andreas Dr.
21230 Homestead Rd 104
Cupertino,
CA 95014 (US)**

(74) Vertreter: **Grossner, Lutz, Dr. et al
Grenzacher Strasse 124
Postfach 3255
CH-4002 Basel (CH)**

Rank Xerox (UK) Business Services
(3.10/3.09/3.3.3)

**Beschreibung**

Die vorliegende Erfindung betrifft die Verwendung von Mischmicellen aus Gallensäuresalzen und Lipiden in pharmazeutischen oder kosmetischen Präparate zur topischen Verabreichung, die gegebenenfalls einen pharmazeutischen oder kosmetischen Wirkstoff enthalten.

Das Dokument WO-A-90/08534 beschreibt ein Verfahren zur Herstellung wässriger Mischmicell-Lösungen. Das Dokument US-A-4 115 313 beschreibt wässrige Emulsionssysteme mit Gallensäuren, die Phospholipide enthalten können.

Als Gallensäuresalze kommen in der vorliegenden Erfindung die in der DE-OS 2 730 570 erwähnten Salze von Gallensäuren bzw. Gallensäurederivaten in Betracht, insbesondere Cholate, Glycocholate, Deoxycholate und Taurocholate, insbesondere die Alkalisalze, wie die Natriumsalze. Besonders bevorzugt ist Na-Glycocholat.

Als Lipide kommen insbesondere Phosphatidylcholine, z.B. natürliche Lecithine oder synthetische Lecithine mit abgewandelten Seitenketten (z.B. solche, die in der europäischen Patentanmeldung A2-0154977 beschrieben sind) in Betracht. Bevorzugt sind natürliche Lecithine wie Ei- oder Soja-Lecithin.

Das Molverhältnis zwischen Lipid und Gallensäuresalz beträgt 0,1:1 bis 2:1. Bevorzugt sind Mischungsverhältnisse von 0,1:1 bis 1,5:1.

Als Wirkstoffe für die Präparate zur erfindungsgemässen Verwendung kommen alle therapeutisch und kosmetisch wirksamen Stoffe in Betracht, die zur Anwendung auf Haut und Schleimhaut einschliesslich des Auges geeignet sind.

Beispiele solcher Wirkstoffe sind Corticosteroide, wie Hydrocortison, Hydrocortisonacetat, Prednisolon, Fluorcortolon, Triamcinolonacetat; Sexualhormone, wie Estriol, Estradiol, Estradiolbenzoat; Antiphlogistika, wie Indometacin, Bufexamac, Salicylsäure, Salicylsäureamid; Immunsuppresiva, wie Cyclosporin A, FK 506 und immunsuppresive Retinoide, wie (all-E)-3,7-Dimethyl-9-(2-trifluormethyl)-6-nonyloxyphenyl-2,4,6,8-nonatetraensäure; Antibiotika, wie Neomycin, Gentamycin, Polymyxin B, Bacitracin, Gramidicin, Tyrothricin, Erythromycin, Clindamycin, Tetracycline, Chloramphenicol, Fusidinsäure, Nitrofural; Antimycotika, wie Tolnaftat, Imidazol-Derivate (z.B. Miconazol, Econazol), Amorolfin, Nystatin, Amphotericin B, Flucytosin, Griseofulvin; Virustatika und Cytostatika, wie Idoxuridin, Tromantadin, Acylovir, Podophyllin, 5-Fluorouracil; Antipsoriatika, wie Anthralin und Psoralene; Retinoide, wie Tretinoin, Isotretinoin, Arotinoide; Sonnenschutzmittel, wie p-Aminobenzoesäure-Derivate, Benzimidazol-Derivate, Zimtsäure-Derivate; Hautpflegemittel, wie Panthenol; Vitamine, wie Tocopherol; Feuchthaltemittel, wie Pyrrolidoncarbonsäure und deren Na-Salz, Milchsäure und deren Na-Salz; durchblutungsfördernde Mittel, wie Nicotinsäure-Derivate und Capsicide. Weitere Beispiele von Wirkstoffen sind Heparin, PAF-Antagonisten, Leukotrienantagonisten, Antihistaminika, Mastzellblocker, Lokalanästhetika, Peptide und Proteine, insbesondere Cytokine, z.B. Interferon und Interleukin.

Beispiel von topischen Applikationsformen sind Lösungen, Lotionen, Sprays, Crèmes, Gele, Salben und Schäume.

Die Herstellung der Präparate kann unter Anwendung von in der Galenik an sich bekannten Technologien erfolgen, wobei der Wirkstoff oder die Wirkstoffe in Form einer wässrigen Lösung von Gallensäuresalz-Lipid-Mischmicellen eingesetzt werden.

Aus diesen Lösungen werden Gele durch Zusatz eines oder mehrerer anionischer (z.B. Carbomer, Carboxymethylcellulosen und deren Salze, Xanthane, Bentonite, Montmorillonite), kationischer (z.B. Polyquaternium) oder nichtionischer Gelbildner erhalten. Vornehmlich werden aber nichtionische Gelbildner wie: Methylcellulose, Hydroxyäthylcellulose, Hydroxypropylcellulose, Hydroxypropylmethylcellulose, Polyvinylpyrrolidon, Polyvinylalkohol und deren Copolymere, z.B. mit Vinylacetat eingesetzt.

Schäume können als äthanolhaltige wässrige MischmicellLösungen in Druckgaspackungen, die etwa 10 Gew.-% Treibgas, z.B. FKW 12/114 (40:60) enthalten, vorliegen bzw. durch Versprühen aus solchen Druckgaspackungen erzeugt werden. Sprays können aus der Lösung mittels konventioneller Zerstäuber erzeugt werden.

Zur Herstellung von Crèmes und Salben wird, z.B. wie in der DE-OS 2730570 beschrieben, eine wässrige micelläre Wirkstofflösung bereitet. Diese Lösung wird in eine konventionelle Crème- oder Salbengrundlage eingearbeitet. Für diesen Zweck ist jede konventionelle Crème- oder Salbengrundlage geeignet. Solche Grundlagen können auf der Basis von bekannten Hilfsstoffen wie Polyäthylenglykolen, Paraffinen, Wachsen, Fetten und fettähnlichen Stoffen aufgebaut sein und als Oel-in-Wasser oder als Wasser-in-Oel-Typ vorliegen.

Die Präparate können Hilfs- und Zusatzstoffe enthalten, insbesondere Penetrationsverstärker, wie ungesättigte Fettsäuren, z.B. Oelsäure; weiterhin konventionelle Konservierungsmitel (z.B. p-Hydroxybenzoesäureester, Benzylalkohol, Phenoxyäthanol, Chlorhexidin-Salze); Antioxidantien (BHT, BHA, Tocopherol,

Ascorbinsäure); Komplexierungsmittel (Na$_2$-EDTA); Puffer (Citronensäure, Phosphorsäure); Feuchthaltemittel (Propylenglykol, Glycerin, Zucker und Zuckeralkohole, z.B. Sorbitol) sowie Wasser und andere Lösungsmittel wie Aethanol, DMSO oder organische Amide.

Die Präparate können auf der Haut, auf Schleimhäuten, z.B. buccal oder nasal als Gel, Lösung, Zerbeisskapsel oder Buccaltablette; am Auge als Crème, Salbe, Gel oder Lösung; und in den Atemwegen als Spray, angewandt werden.

Mischmicell-Lösungen von pharmazeutischen Wirkstoffen können auch in transdermalen Applikationssystemen Anwendung finden. Die Präparate umfassen daher auch transdermale Präparate, in denen die Kombination von Wirkstoff, Gallensäure(salz) und Lipid als Lösung oder in trockener, z.B. in lyophilisierter Form enthalten ist.

Die Präparate besitzen im Vergleich zu konventionellen Formulierungen vorteilhafte Eigenschaften, beispielsweise eine bessere Penetration des Wirkstoffs, und dessen bessere Verteilung in der Haut, sowohl für hydrophile als auch für lipophile Wirkstoffe. Bei letzteren besteht zusätzlich der Vorteil, dass auf den Einsatz von schlecht verträglichen organischen Lösungsmitteln verzichtet werden kann. Die Präparate zeichnen sich z.B. gegenüber liposomalen Formulierungen durch bessere physikalische Stabilität und technisch weniger aufwendige Herstellung aus.

Die Präparate besitzen eine gute Verträglichkeit auf der Haut, auf Schleimhäuten und am Auge.

Primäre Augen- und Hautreizungstests mit wirkstofffreien Mischmicell-Formulierungen wurden gemäss der OECD-Guidelines durchgeführt. Die Präparate erweisen sich alle als "non-irritating" und heben sich dadurch gebräuchlicher Solubilisaten (z.B. mit Natriumlaurylsulfat) ab.

Es wurde gefunden, dass topische Formulierungen, die ausser Gallensäuresalz/Lipid-Mischmicellen keinen pharmazeutischen Wirkstoff enthalten, eine antimykotische Wirkung aufweisen. Die Erfindung betrifft daher auch die Verwendung von Mischmicellen aus Gallensäuresalzen und Lipiden mit einem Molverhältnis Lipid: Gallensäuresalz von 0,1:1 bis 2:1 als Wirkstoffe bei der Herstellung von Präparaten zur topischen Behandlung von Mykosen.

Die Erfindung wird durch die nachstehenden Beispiele weiter erläutert.

Alle Operationen werden unter Inertgas durchgeführt; die Lösungsmittel werden durch Einleiten von Inertgas von Sauerstoff befreit.

Beispiel 1

In 10,0 g Aethanol (oder einem anderen geeigneten organischen Lösungsmittel) werden 1,75 g Glycocholsäure, 1,50 g Soja-Lecithin, 0,278 g Amorolfin-HCl und 1,0 g Benzylalkohol nacheinander bei ca. 45-50°C klar gelöst (Lösung A). 3,75 ml einer 1N NaOH-Lösung werden zu 80 ml entsalztem Wasser gegeben. Unter Rühren wird diese Lösung der Lösung A zugefügt und die Mischung mit Wasser auf 100,0 g ergänzt.

Beispiel 2

1,75 g Glycocholsäure werden in ca. 50 g entsalztem Wasser suspendiert und durch Zugabe von NaOH gelöst. Nach Einstellen eines pH-Wertes von 6,0 werden nacheinander 1,50 g Soja-Lecithin, 0,278 g Amorolfin-HCl und 1,0 g Benzylalkohol gelöst (Lösung A). Durch Ultraschall und/oder Erwärmen lässt sich der Lösungsvorgang beschleunigen. 1,60 g Methylcellulose 4000 cP werden in 5,0 g Propylenglykol suspendiert und der Lösung A unter Rühren zugefügt. Mit Wasser wird auf 100,0 g ergänzt und das Gel bis zum vollständigen Ausquellen der Methylcellulose bei 5°C langsam gerührt.

Beispiel 3

In Analogie zu obengenannten Beispielen können Gele zur topischen Verabreichung von Amorolfin mit folgenden Zusammensetzungen hergestellt werden:

|                     | 3a    | 3b    | 3c    |     |
|---------------------|-------|-------|-------|-----|
| Amorolfin HCl       | 0,278 | 0,278 | 0,278 | g   |
| Benzylalkohol       | 1,00  | 1,00  | 1,00  | g   |
| Propylenglykol      | 5,00  | 5,00  | 5,00  | g   |
| Lecithin            | 1,50  | 1,5   | 7,45  | g   |
| Glycocholsäure      | 1,75  | 1,75  | 5,38  | g   |
| NaOH ad pH 6,0-6,2  | q.s.  | q.s.  | q.s.  |     |
| Methylcellulose     | 1,6   | -     | -     | g   |
| Hydroxyäthylcellulose | -   | 1,80  | 1,80  | g   |
| Aethanol            | -     | 10,0  | -     | g   |
| Wasser ad           | 100,0 | 100,0 | 100,0 | g   |

Wie ein Vergleich der Penetrationsdaten zeigt, werden sehr viel bessere Wirkstoffkonzentrationen sowohl in der Hornhaut als auch in den tieferen Hautschichten mit den Gallensäuresalz/Lecithin-Systemen erreicht als bei Einsatz von klassischen Crèmes.

| Formu-lierung | Dosis | Zeit (h) | Haut-oberfl. | Str. corneum | Rest-haut | Kammer-flüssigkeit |           |
|---------------|-------|----------|--------------|--------------|-----------|--------------------|-----------|
| Gel von Bsp. 3a | 0,25% | 16 | 9,99 | 1,74 | 3,22 | 0,05 | ($\mu$g/cm$^2$) |
|               |       |          | 66,6 | 11,6 | 21,5 | 0,3  | (%)       |
| Gel von Bsp. 3b | 0,25% | 16 | 5,04 | 3,71 | 6,21 | 0,04 | ($\mu$g/cm$^2$) |
|               |       |          | 33,6 | 24,7 | 41,4 | 0,3  | (%)       |
| Gel von Bsp. 3c | 0,25% | 16 | 11,92 | 1,41 | 1,65 | 0,02 | ($\mu$g/cm$^2$) |
|               |       |          | 79,4 | 9,4 | 11,0 | 0,1  | (%)       |
| Crème         | 0,25% | 16 | 14,19 | 0,32 | 0,48 | 0,01 | ($\mu$g/cm$^2$) |
|               |       |          | 94,6 | 2,1 | 3,2 | 0,1  | (%)       |

4

Beispiel 4

Eine Mischmicell-Lösung von Isotretinoin kann folgende Zusammensetzung aufweisen:

| | |
|---|---|
| Isotretinoin | 50 mg |
| dl-$\alpha$-Tocopherol | 10 mg |
| $Na_2$-EDTA | 30 mg |
| Lecithin | 16,9 g |
| Glycocholsäure | 8,85 g |
| Benzylalkohol | 1,0 g |
| NaOH ad pH 6 | q.s. |
| Wasser ad | 100,0 g |

Diese Mischmicell-Lösung kann analog Beispiel 2 zu einem Gel verarbeitet werden. Das Gel kann zur topischen Behandlung z.B. von Akne angewandt werden.

Beispiel 5

Eine Mischmicell-Lösung von Tretinoin kann folgende Zusammensetzung aufweisen:

| | |
|---|---|
| Tretinoin | 20 mg |
| dl-$\alpha$Tocopherol | 10,0 mg |
| Lecithin | 16,9 g |
| Glycocholsäure | 8,85 g |
| NaOH ad pH 6 | q.s. |
| Benzylalkohol | 1,0 g |
| Wasser ad | 100,0 ml |

Diese Mischmicell-Lösung kann wie in Beispiel 2 beschrieben zu einem Gel verarbeitet werden. Das Gel kann auch zur topischen Behandlung z.B. von Akne angewandt werden.

Beispiel 6

Eine Mischmicell-Lösung zur topischen Anwendung bei Psoriasis kann folgende Zusammensetzung aufweisen:

| | |
|---|---|
| Methyl-p-[2-(5,6,7,8-tetrahydro-5,5,8,8-tetramethyl-2-naphthyl)propyl]phenylsulfon | 0,05 g |
| Lecithin | 16,90 g |
| Glycocholsäure | 8,85 g |
| NaOH ad pH 6,0 | q.s. |
| Benzylalkohol | 1,00 g |
| Wasser ad | 100,00 g |

5

Die Lösung kann in Analogie zu Beispiel 2 in ein Gel übergeführt werden.

Beispiel 7

Mischmicell-Lösungen zur Herstellung eines Hydrocortison-Sprays können folgende Zusammensetzung aufweisen:

|  | (a) | (b) |
| --- | --- | --- |
| Hydrocortison | 0,25 | 0,50 g |
| Lecithin | 8,00 | 10,58 g |
| Glycocholsäure | 5,00 | 8,06 g |
| NaOH ad pH 6,0 | q.s. | q.s. |
| Benzylalkohol | 1,00 | 1,00 g |
| Wasser ad | 100,00 | 100,00 g |

Mit Mischmicell-Lösungen, die als Spray eingesetzt werden können, lassen sich bessere Gewebespiegel an Hydrocortison in der Haut erreichen als bei Applikation von liposomalen Trägern:

| Formulierung | Dosis | Zeit (h) | Hautoberfl. | Str. corneum | Resthaut | Kammerflüssigkeit | |
| --- | --- | --- | --- | --- | --- | --- | --- |
| Mischmicelllösung (a) von Bsp. 7 | 0,25% | 6 | 12,39<br>82,6 | 1,48<br>9,9 | 1,04<br>6,9 | 0,09<br>0,6 | ($\mu g/cm^2$)<br>(%) |
| Liposomen sol. | 0,25% | 6 | 13,46<br>89,7 | 0,68<br>4,5 | 0,84<br>5,6 | 0,02<br>0,1 | ($\mu g/cm^2$)<br>(%) |

Die Hydrocortison-Liposomen wurden analog zu einer Vorschrift von Wohlrab und Lasch (Dermatologica 174, 18(1987)) bereitet.

Die Lösungen 7a und 7b können als Spray zur topischen Applikation bei entzündlichen Hauterkrankungen verwendet werden.

Beispiel 8

Eine Mischmicell-Lösung von Panthenol kann folgende Zusammensetzung aufweisen:

| D-Panthenol | 0,50 g |
| --- | --- |
| Lecithin | 8,00 g |
| Glycocholsäure | 5,00 g |
| NaOH ad pH 6,0 | q.s. |
| Benzylalkohol | 1,00 g |
| Wasser ad | 100,00 g |

Die Lösung kann als Spray zur Behandlung von Hautschäden angewandt werden.

Wie ein Vergleich der in vitro-Penetrationsdaten zeigt, werden mit einer Mischmicell-Lösung deutlich höhere Panthenol-Konzentrationen in der Haut erreicht als mit einer konventionellen Salbe:

| Formu-lierung | Panthenol-Gehalt | Zeit (h) | Haut-oberfl. | Str. corneum | Rest-haut | Kammer-flüssigkeit | |
|---|---|---|---|---|---|---|---|
| Lösung von Bsp. 8 | 0,50% | 6 | 222,6 74,2 | 51,99 17,3 | 22,44 7,5 | 2,9 1,9 | $(\mu g/cm^2)$ (%) |
| konventio-nelle Salbe | 0,50% | 6 | 300,9 96,4 | 7,70 25 | 2,98 1,0 | 0,43 0,1 | $(\mu g/cm^2)$ (%) |

Beispiel 9

Cyclosporin A Mischmicell-Lösungen können folgende Zusammensetzung aufweisen:

| Cyclosporin A | 1,7 - 3,7 mg |
|---|---|
| Lecithin | 30,8 - 92,4 mg |
| Na-Glycohalat | 19,5 - 58,5 mg |
| Wasser oder Puffer ad | 1,0 ml |

Lecithin (Lipoid E PG, Lipoid KG), Natriumglycocholat und Cyclosporin werden in einem Rundkolben in 5 ml Chloroform/Methanol (1:1, V/V) gelöst. Der nach Abdampfen der organischen Lösungsmittel (40°C) resultierende Film wird in 1 ml Wasser dispergiert und mit 1N HCl auf pH 6,0 ± 0,1 eingestellt. Zur Hydratisierung des Films können auch Puffer-Lösungen wie Phosphatpuffer oder Polyalkohol-Lösungen z.B. Mannitlösungen, eingesetzt werden.

Beispiel 10

Eine Mischmicell-Lösung von Vitamin A-Palmitat kann folgende Zusammensetzung aufweisen.

| Vitamin A-Palmitat | 5000 IE |
|---|---|
| Glycocholsäure | 23,6 mg |
| Lecithin | 35,0 mg |
| Propylenglykol | 50,0 mg |
| Aethanol | 50,0 mg |
| NaOH ad pH 6,0 | q.s. mg |
| Benzylalkohol | 10,0 mg |
| Wasser | ad 1000,0 mg |

In dem Gemisch aus Propylenglykol, Aethanol und Benzylalkohol werden Glycocholsäure, Lecithin und das Tocopherol-stabilisierte Vitamin A-Palmitat klar gelöst. Der pH-Wert der Lösung wird mit NaOH auf 6,0 ± 0,1 eingestellt. Anschliessend wird mit Wasser auf 1000 mg ergänzt.

Beispiel 11

Tocopherolacetat-Mischmicell-Lösungen zur topischen Anwendung können folgende Zusammensetzung aufweisen:

11a) Formulierungen mit Na-Glycocholat als Detergens

| | | a1 | a2 |
|---|---|---|---|
| dl-α-Tocopherolacetat | | 2,0 | 5,0 g |
| dl-α-Tocopherol | | 0,05 | 0,05 g |
| Na-Glycocholat | | 2,0 - 3,39 | 5,0 g |
| Lecithin | | 3,0 - 5,0 | 10,0 g |
| Propylenglykol | | 5,0 | 5,0 g |
| Aethanol | | 5,0 | 5,0 g |
| Benzylalkohol | | 1,0 | 1,0 g |
| Wasser | ad | 100,0 | 100,0 g |

Aethanol, Tocopherol, Tocopherolacetat, Propylenglykol, Benzylalkohol, Lecithin und Na-Glycocholat werden nacheinander zugefügt und unter leichtem Erwärmen klar gelöst. Danach wird mit Wasser versetzt und der pH-Wert auf 6,0 eingestellt.

Formulierungen von Tocopherolacetat (2%) auf der Basis von Lecithin-Gallensalz-Mischmicellen zeichnen sich gegenüber konventionellen Tocopherolacetat-Solubilisaten dadurch aus, dass nur physiologische Solubilisatoren eingesetzt werden. Dennoch ist die in-vitro Hautpenetration aus der Mischmicell-Lösung leicht besser als aus einem analogen, konventionellen Solubilisat mit 10% PEG-36 Castor Oil.

Nach einer Penetrationszeit von 6 h wurde folgende Verteilung in der Schweinehaut bestimmt:

| Formulierung mit 2% Tocopherolacetat | Haut- oberfl. | Str. corneum | Rest- haut |
|---|---|---|---|
| Mischmicell-Lösung 1) | 88,8% | 8,9% | 2,3% |
| konventionelles Solubilisat | 90,4% | 7,8% | 1,8% |

1) 3,5% Lecithin und 2,47% Na-Glyocholat

11b) Formulierungen mit Na-Cholat als Detergens

| | | |
|---|---|---|
| dl-α-Tocopherolacetat | | 2,0 g |
| dl-α-Tocopherol | | 0,05 g |
| Na-Cholat | | 2,5 g |
| Lecithin | | 3,0 g |
| Propylenglykol | | 5,0 g |
| Aethanol | | 5, 0g |
| Wasser | ad | 100,0 g |

Eine Lösung von Tocopherolacetat und Tocopherol (2,0 + 0,05 g) in Aethanol und das in Chloroform/Methanol (1:1) gelöste Lecithin (3,0 g) werden gemischt und im Vakuum zu einem Film getrocknet. Der Film wird in 5,0 g Aethanol und 5,0 g Propylenglykol in der Wärme gelöst Anschliessend wird diese Lösung mit einer wässrigen Lösung von 2,0 g Na-Cholat versetzt, geschüttelt und erwärmt. Mit 1N HCl wird der pH-Wert auf 6,0 ± 0,1 eingestellt und mit Wasser auf 100,0 g ergänzt. Sodann wird bei Raumtemperatur solange gerührt, bis eine klare Lösung entstanden ist (ca. 18 Stunden).

**Patentansprüche**

1. Verwendung von Mischmicellen aus Gallensäuresalzen und Lipiden mit einem Molverhältnis Lipid: Gallensäuresalz von 0,1:1 bis 2:1 in pharmazeutischen oder kosmetischen Präparaten zur topischen Verabreichung.

2. Verwendung nach Anspruch 1, dadurch gekennzeichnet, dass das Salz der Gallensäure Na-Glycocholat und das Lipid Phosphatidylcholin, insbesondere natürliches Lecithin ist.

3. Verwendung nach den Ansprüchen 1-3, zur topischen Verabreichung eines Retinoids.

4. Verwendung nach den Ansprüchen 1-3, zur topischen Verabreichung eines Antimykotikums.

5. Verwendung nach den Ansprüchen 1-3, zur topischen Verabreichung von Pantenol.

6. Verwendung nach den Ansprüchen 1-6 in Form einer Lösung oder eines Gels.

7. Verwendung von Mischmicellen aus Gallensäuresalzen und Lipiden mit einem Molverhältnis Lipid: Gallensäuresalz von 0,1:1 bis 2:1 als Wirkstoffe bei der Herstellung von Präparaten zur topischen Behandlung von Mykosen.

**Claims**

1. The use of mixed micelles from cholanic acid salts and lipids with a molar ratio lipid:cholanic acid salt of 0.1:1 to 2:1 in pharmaceutical or cosmetic preparations for topical administration.

2. The use according to claim 1, characterized in that the cholanic acid salt is Na glycocholate and the lipid is phosphatidylcholine, especially natural lecithin.

3. The use according to claims 1-3 for the topical administration of a retinoid.

4. The use according to claims 1-3 for the topical administration of an antimycotic.

5. The use according to claims 1-3 for the topical administration of pantenol.

**6.** The use according to claims 1-6 in the form of a solution or a gel.

**7.** The use of mixed micelles from cholanic acid salts and lipids with a molar ratio lipid:cholanic acid salt of 0.1:1 to 2:1 as active substances in the manufacture of preparations for the topical treatment of mycoses.

**Revendications**

**1.** Utilisation de micelles mixtes de sels d'acide biliaire et de lipides selon un rapport en moles du lipide au sel d'acide biliaire de 0,1:1 à 2:1 dans des préparations pharmaceutiques ou cosmétiques pour appli-cation topique.

**2.** Utilisation selon la revendication 1, caractéri-sée en ce que le sel d'acide biliaire est le glycocholate de Na et le lipide est une phosphatidylcholine, notamment une lécithine naturelle.

**3.** Utilisation selon les revendications 1 à 3, pour application topique d'un rétinoïde.

**4.** Utilisation selon les revendications 1 à 3, pour application topique d'un antimycotique.

**5.** Utilisation selon les revendications 1 à 3, pour application topique de panthénol.

**6.** Utilisation selon les revendications 1 à 5, sous forme d'une solution ou d'un gel.

**7.** Utilisation de micelles mixtes de sels d'acide biliaire et de lipides présentant un rapport en moles du lipide au sel d'acide biliaire de 0,1:1 à 2:1, comme principes actifs lors de la fabrication de préparations pour le traitement topique des mycoses.